# EUROPÄISCHE PATENTSCHRIFT

(11) **EP 0 727 201 B1**
(45) Veröffentlichungstag und Bekanntmachung des Hinweises auf die Patenterteilung: **27.11.1996**
(21) Anmeldenummer: 95111664.9
(22) Anmeldetag: 25.07.1995
(51) Int. Cl.: A61K 7/09

(54) **Mittel zur dauerhaften Verformung von menschlichen Haaren**
Composition for the permanent waving of human hair
Composition pour la déformation permanente des cheveux

(30) Priorität: 12.08.1994 DE 4428574
(43) Veröffentlichungstag der Anmeldung: 21.08.1996
(73) Patentinhaber: GOLDWELL GmbH, D-64297 Darmstadt (DE)
(72) Erfinder: Glauder, Jan, Dr., D-64297 Darmstadt (DE)

(56) Entgegenhaltungen:
- CH-A- 301 601
- DE-A- 2 929 865
- DE-C- 4 304 828
- US-A- 4 459 284

## Beschreibung

Die vorliegende Erfindung betrifft ein Dauerwellmittel mit guter Wellwirksamkeit, das jedoch gleichwohl eine haarschonende Behandlung gewährleistet.

Die Dauerwellung erfolgt bekanntlich in zwei Behandlungsschritten, der reduktiven Spaltung der Cystin-Disulfidbrücken des Haares durch Einwirkung eines Reduktionsmittels, und die anschließende Neutralisierung bzw. Fixierung durch Aufbringung eines Oxidationsmittels, wodurch die Cystin-Disulfidbrücken wiederhergestellt werden.

Das überwiegend eingesetzte Reduktionsmittel ist auch heute noch die Thioglykolsäure, auch in Form ihrer Salze, insbesondere des Ammoniumsalzes, obwohl zahlreiche andere Thioverbindungen für diesen Zweck vorgeschlagen wurden, die sich jedoch in der Praxis zumeist nicht durchgesetzt haben.

Die Thioglykolat enthaltenden Zusammensetzungen werden üblicherweise bei einem pH-Wert zwischen 8 und 10, insbesondere 8,5 und 9,5, eingesetzt, was bei wiederholter, zeitlich nahe zusammenliegender Anwendungen zu Haarschädigungen führen kann.

Man hat bereits versucht, diese Nachteile durch die Schaffung sogenannter "saurer Dauerwellmittel" zu überwinden, deren Anwendungs-pH-Wert bei etwa 6,8 bis 7,8, d.h. um den Neutralpunkt herum, liegt. Der in diesen Zusammensetzungen meistgenutzte reduzierende Wirkstoff ist der Thioglykolsäuremonoglycerinester. Es hat sich jedoch gezeigt, daß diese Substanz bei manchen Benutzerinnen hautreizend, insbesondere sensibilisierend, wirken kann, so daß auch diese Problemlösung nicht optimal ist.

Eine Lösung dieses Problems ist aus der DE-C 43 04 828 bekannt: der Einsatz eines Mittels, das Thioglykolsäure bzw. ein Salz derselben enthält und unmittelbar vor der Anwendung durch Vermischen von zwei bis dahin getrennt gehaltenen Zusammensetzungen hergestellt wird, wobei die eine Zusammensetzung (A) mindestens ein Aminosäurehydrochlorid und mindestens ein Polyol bzw. dessen Methyl- und Ethylether auf wäßriger Grundlage enthält, und einen pH-Wert zwischen 4,5 und 6,5, vorzugsweise zwischen 5 und 6, aufweist, und eine Zusammensetzung (B), die als Alkalisierungsmittel Ammoniumhydrogencarbonat, Ammoniumcarbonat und/oder Ammoniumcarbamat enthält und einen pH-Wert zwischen etwa 8 und 9,5 aufweist und das beim Vermischen beider Zusammensetzungen erhaltene, gebrauchsfertige Mittel (AB) einen pH-Wert zwischen 7 und 8 besitzt.

Das bevorzugte Aminosäurehydrochlorid, das gleichzeitig auch noch einen Beitrag zur Wellwirkung leistet, ist das Cysteinhydrochlorid.

Es ist auch möglich, dieses durch ein anderes Aminosäurehydrochlorid, beispielsweise Glycinhydrochlorid, auszutauschen, jedoch wird damit eine gewisse wenn auch geringe Verschlechterung der Wellwirksamkeit in Kauf genommen.

Es hat sich jedoch gezeigt, daß Cysteinhydrochlorid enthaltende Zusammensetzungen mit bestimmten Kunststoffmaterialien, die für die Herstellung der für die Lagerung solcher Produkte vorgesehenen Zweikammer-Behältnisse eingesetzt werden, nicht voll verträglich sind, sondern zu einer Gelbfärbung derselben führen, was zumindest aus ästhetischen Gründen unerwünscht ist.

Bei Anwendung anderer, keine SH-Gruppen aufweisender Aminosäuren bzw. deren Hydrochloriden tritt dieser Effekt jedoch nicht auf, was jedoch durch eine Verringerung der Wellwirkung erkauft wird.

Es wurde nun gefunden, daß der leichte Abfall der Weilwirksamkeit beim Austausch des Cysteinhydrochlorids durch andere Aminosäuren bzw. Aminosäurehydrochloride dann verhindert werden kann, wenn die Zusammensetzung einen geringen Anteil, mindestens 1 ppm, vorzugsweise 1 bis 100, insbesondere 2 bis 50, vor allem 3 bis 10, ppm Eisen(II)ionen, berechnet auf die Gesamtzusammensetzung, enthält.

Gegenstand der Erfindung ist also ein Mittel zur dauerhaften Verformung von menschlichen Haaren, das mindestens eine reduzierend wirkende Thioverbindung, vorzugsweise Thioglykolsäure und/oder ein Salz derselben, enthält und aus zwei bis zur Anwendung voneinander getrennt gehaltenen Zusammensetzungen besteht, wobei die eine Zusammensetzung (A) mindestens eine Aminosäure bzw. deren Hydrochlorid, ausgenommen Cystein, und gegebenenfalls mindestens ein Polyol bzw. dessen Methyl- oder Ethylether in wäßriger Lösung enthält und einen pH-Wert zwischen 3 und 6,5, insbesondere 4,5 und 6,5, vorzugsweise zwischen 5,0 und 6,0, aufweist, und die zweite, davon getrennt gehaltene Zusammensetzung (B) ein Alkalisierungsmittel, insbesondere Ammoniumhydrogencarbonat, Ammoniumcarbonat und/oder Ammoniumcarbamat, enthält, wobei der pH-Wert zwischen etwa 8 und 12, vorzugsweise 8 und 9,5, insbesondere 8,1 und 8,6, liegt, und beide Zusammensetzungen unmittelbar vor dem Aufbringen auf das menschliche Haar vermischt werden, und die dadurch hergestellte gebrauchsfertige Zusammensetzung (AB) einen pH-Wert zwischen 7 und 8 aufweist, das dadurch gekennzeichnet ist, daß die mindestens eine Aminosäure bzw. deren Hydrochlorid enthaltende Zusammensetzung (A) Eisen(II)ionen enthält.

Durch diese Maßnahme kann gleichzeitig eine Verfärbung des Kunststoffmaterials des Zweikammer-Behälters, der die Wirksubstanz enthält, verhindert und darüber hinaus eine optimale Wellwirkung des Produktes sichergestellt werden.

Geeignete Aminosäuren bzw. Aminosäurehydrochloride sind die in der bereits erwähnten DE-C 43 04 828 beschriebenen, deren Anteil vorzugsweise bei etwa 0,25 bis etwa 5 Gew.-%, bezogen auf die reine Aminosäure und die Gesamtzusammensetzung des Mittels, liegt.

Bevorzugt wird Glycin, entweder als Aminosäure oder als Hydrochlorid eingesetzt, jedoch sind beispielsweise auch Alanin, Valin, Leucin, Isoleucin, Prolin, Tryptophan, Phenylalanin, Methionin, Serin, Tyrosin, Threonin, Asparagin, Glutamin oder Histidin geeignet.

Die Zusammensetzung enthält, wie bereits ausgeführt, mindestens eine reduzierend wirkende Thioverbindung, vorzugsweise Thioglykolsäure und/oder deren Ammoniumsalz, jedoch können auch weitere Salze, beispielsweise Natriumthioglykolat oder Aminthioglykolate, verwendet werden.

Die Mit-Verwendung weiterer wellwirksamer Thioverbindungen, wie sie an sich bekannt sind, z. B. Thiomilchsäure, ist möglich und gegebenenfalls zweckmäßig, jedoch nicht zwingend erforderlich.

Vorzugsweise werden die Thioglykolsäure bzw. deren Salze in der Zusammensetzung (A) eingesetzt.

Der Anteil an Thioverbindungen einschließlich Thioglykolsäure in den erfindungsgemäßen Zusammensetzungen liegt zwischen etwa 4 und etwa 12 Gew.-%, vorzugsweise etwa 6 bis 9 Gew.-%, jeweils berechnet auf die Thioglykolsäure, bezogen auf die Gesamtzusammensetzung des gebrauchsfertigen Mittels (Zusammensetzung A + B).

Der dritte essentielle Bestandteil in den erfindungsgemäßen Zusammensetzungen ist eine Eisen(II)ionen liefernde Substanz in einer Menge von 1 bis 100, insbesondere 2 bis 50, vorzugsweise 3 bis 10 ppm, berechnet auf die die Aminosäure bzw. deren Hydrochlorid enthaltende Teilzusammensetzung (A).

Geeignet sind alle bekannten, in kosmetischen Mitteln einsetzbaren anorganischen und organischen Eisen(II)salze, insbesondere FeSO₄, FeCl₂, Eisen(II)gluconat und Eisen(II)oxalat.

Zusätzlich bevorzugt mitverwendete Komponenten sind Polyole bzw. deren Ether. Als solche werden insbesondere 1,2- oder 1,3-Propandiol, 1,2-, 1,3- oder 1,4-Butandiol sowie deren Methyl- und Ethylether, vorzugsweise 1-Methoxypropanol(-2), Dipropylenglykolmonomethylether und Glycerin eingesetzt.

Die bevorzugte Menge liegt bei etwa 1 bis 15 Gew.-% der Gesamtzusammensetzung (Zusammensetzungen A + B), vorzugsweise bei etwa 2 bis 10 Gew.-%.

Die von der Zusammensetzung (A) bis zur Anwendung getrennt gehaltene Zusammensetzung (B) enthält mindestens ein Alkalisierungsmittel wie Ammoniumhydrogencarbonat, Ammoniumcarbonat und/oder Ammoniumcarbamat, vorzugsweise in einer Menge zwischen etwa 1 und etwa 10 Gew.-%, bevorzugt 2 bis etwa 7, insbesondere etwa 2,5 bis 6 Gew.-%, berechnet auf die Gesamtzusammensetzung (A) + (B).

Die erfindungsgemäßen Dauerwellmittel können alle für diesen Zweck vorgeschlagenen und eingesetzten Zusatzstoffe enthalten.

Zur Vermeidung von Wiederholungen wird hierzu auf den einschlägig bekannten Stand der Technik und dessen Zusammenfassungen, beispielsweise in "Ullmann's Encyclopaedia of Industrial Chemistry", Vol.A12 (1986), S.588 bis 591, sowie auf die Monographie von K.Schrader, "Grundlagen und Rezepturen der Kosmetika", 2. Aufl. (1989, Hüthig Buchverlag), S.823 bis 840, verwiesen.

Die Fixierung der durch Anwendung der erfindungsgemäßen Dauerwell-Zusammensetzungen verformten Haare erfolgt durch die ebenfalls bekannte und übliche Neutralisation mittels Wasserstoffperoxid oder Alkalibromaten.

Die getrennte Unterbringung der beiden Zusammensetzungen gemäß der Erfindung bis zur Anwendung des Mittels geschieht vorzugsweise in den seit langem bekannten Zweikammer-Behältern, deren Trennwand bei der Anwendung durch geeignete Mittel zerstört wird.

Solche Zweikammer-Behältnisse, die sich insbesondere für die erfindungsgemäßen Zusammensetzungen eignen, sind beispielsweise in den deutschen Offenlegungsschriften Nr. 35 28 525, 36 06 003, 38 12 343 und 38 37 595 beschrieben.

Das folgende Beispiel dient der Illustration der Erfindung.

### Zusammensetzung A

| | |
|---|---|
| Ammoniumthioglykolat (70%-ig) | 18,25 (g) |
| Thiomilchsäure | 2,00 |
| Glycin | 1,30 |
| 1,2-Propandiol | 0,50 |
| FeSO₄-Lösung (0,01%-ig) | 0,30 |
| Ammoniak (25%-ig) | 1,35 |
| Wasser | 4,10 |
| pH-Wert: 5,7 | |

### Zusammensetzung B

| | |
|---|---|
| Ammoniumhydrogencarbonat | 4,55 (g) |
| Nichtionischer Emulgator (PEG-35 Castor Oil) | 0,40 |
| Ethoxyliertes Alkylphenol (80%-ig, Nonoxynol-23) | 0,40 |
| Polydimethyldiallylammoniumchlorid (Polyquaternium-6) | 0,30 |
| Parfum | 0,40 |
| Isopropylalkohol | 1,50 |
| Trübungsmittel | 0,10 |
| Ethoxydiglykol | 2,00 |
| Ammoniak (25%-ig) | 0,70 |
| Wasser | 61,40 |
| pH-Wert: 8,3 | |

25 g Zusammensetzung (A) und 65 g Zusammensetzung (B) wurden in ein aus co-extrudiertem Polyethylen/Polyamid/Polymaleinsäure bestehendes Zweikammer-Behältnis verpackt.

Nach sechsmonatiger Lagerzeit erfolgte keine Verfärbung des Kunststoffs, während bei Anwendung einer Cysteinhydrochlorid.H₂O anstelle von Glycin/FeSO₄ enthaltenden Zusammensetzung die Behälterwandung gelb gefärbt war.

Das Dauerwellergebnis der vereinigten Zusammensetzung (AB), deren pH-Wert 7,35 betrug, unterschied sich nicht von demjenigen einer Cysteinhydrochlorid enthaltenden, aus der DE-C 43 04 828 bekannten Well-Lösung.

## Patentansprüche

1. Mittel zur dauerhaften Verformung von menschlichen Haaren, das mindestens eine reduzierend wirkende Thioverbindung, insbesondere Thioglykolsäure und/oder ein Salz derselben, enthält und aus zwei bis zur Anwendung voneinander getrennt gehaltenen Zusammensetzungen besteht, dadurch gekennzeichnet, daß die eine Zusammensetzung (A) mindestens eine Aminosäure bzw. deren Hydrochlorid, ausgenommen Cystein, und mindestens ein geringe Mengen, mindestens 1 ppm, Eisen(II)ionen lieferndes Eisen(II)salz und gegebenenfalls ein Polyol bzw. dessen Methyl- oder Ethylether in wäßriger Lösung enthält, und einen pH-Wert zwischen 3,0 und 6,5 aufweist, und die zweite, davon getrennt gehaltene Zusammensetzung (B) mindestens ein Alkalisierungsmittel aus der Gruppe Ammoniumhydrogencarbonat, Ammoniumcarbonat und/oder Ammoniumcarbamat enthält, wobei der pH-Wert zwischen 8 und 12 liegt, und beide Zusammensetzungen unmittelbar vor der Anwendung auf menschliches Haar vermischt werden, und die dadurch hergestellte gebrauchsfertige Zusammensetzung (AB) einen pH-Wert zwischen 7 und 8 aufweist.

2. Mittel nach Anspruch 1, dadurch gekennzeichnet, daß die Zusammensetzung (A) einen pH-Wert zwischen 4,5 und 6,5 aufweist.

3. Mittel nach Anspruch 1, dadurch gekennzeichnet, daß die Zusammensetzung (B) einen pH-Wert zwischen 8 und 9,5 aufweist.

4. Mittel nach einem der vorhergehenden Ansprüche, dadurch gekennzeichnet, daß die nach dem Vermischen der Zusammensetzungen (A) und (B) erhaltene, auf das Haar aufzubringende gebrauchsfertige Zusammensetzung (AB) einen pH-Wert zwischen 7,3 und 7,7 aufweist.

5. Mittel nach einem der vorhergehenden Ansprüche, dadurch gekennzeichnet, daß es als Aminosäure Glycin enthält.

6. Mittel nach einem oder mehreren der Ansprüche 1 bis 5, dadurch gekennzeichnet, daß es mindestens ein Eisen(II)salz in einer solchen Konzentration enthält, daß der Gehalt an Eisen(II)ionen zwischen 1 und 100 ppm liegt.

7. Mittel nach Anspruch 6, dadurch gekennzeichnet, daß der Gehalt an Eisen(II)ionen zwischen 2 und 50 ppm liegt.

8. Mittel nach Anspruch 7, dadurch gekennzeichnet, daß der Gehalt an Eisen(II)ionen bei 3 bis 10 ppm liegt.

## Claims

1. Composition for permanent waving of human hair comprising at least one active reducing thio compound, particularly thioglycolic acid and (or) a salt thereof, consisting of two compositions which are kept separate until application onto the hair, wherein one aqueous composition (A) comprises at least one amino acid or the hydrochloride thereof, except cysteine, and at least one ferrous salt releasing small quantities of ferrous ions, and optionally a polyol or the methyl or ethyl ethers thereof, having a pH-value between 3.0 and 6.5, and a second composition (B), which is kept separate until application, comprising at least one alkalizing agent selected from the group of ammonium hydrogen carbonate, ammonium carbonate and (or) ammonium carbamate, having a pH-value between 8 and 12; whereof both compositions are mixed immediately before application onto human hair and the ready-to-use composition (AB) thus prepared having a pH-value between 7 and 8.

2. Composition according to claim 1, wherein composition (A) has a pH-value between 4.5 and 6.5.

3. Composition according to claim 1, wherein composition (B) has a pH-value between 8 and 9.5.

4. Composition according to one of the preceding claims, wherein the ready-to-use composition (AB), which is obtained after mixing composition (A) with composition (B) to be applied onto the hair, has a pH-value between 7.3 and 7.7.

5. Composition according to one of the preceding claims, comprising glycine as amino acid.

6. Composition according to one or more of the claims 1 to 5, comprising at least one ferrous salt in such a concentration that the content of ferrous ions is between 1 and 100 ppm.

7. Composition according to claim 6, wherein the concentration of ferrous ions is between 2 and 50 ppm.

8. Composition according to claim 7, wherein the concentration of ferrous ions is between 3 to 10 ppm.

## Revendications

1. Produit pour la mise en forme permanente des cheveux humains, qui contient au moins un composé thio à effet réducteur, en particulier de l'acide thioglycolique et/ou un sel de celui-ci, et consiste en deux compositions maintenues séparées l'une de l'autre jusgu'à l'emploi, caractérisé en ce qu'une composition (A) contient en solution aqueuse au moins un aminoacide ou son chlorhydrate, à l'exception de la cystéine, et une faible quantité, d'au moins un sel ferreux fournissant des ions ferreux, et éventuellement un polyol ou un éther méthylique ou éthylique de celui-ci, et présente une valeur de pH comprise entre 3,0 et 6,5, et la seconde composition (B), maintenue séparée de celle-ci, contient au moins un agent d'alcalinisation choisi parmi le groupe hydrogénocarbonate d'ammonium, carbonate d'ammonium et/ou carbamate d'ammonium, la valeur de pH étant comprise entre 8 et 12, et les deux compositions sont mélangées immédiatement avant l'emploi sur le cheveu humain, et la composition prête à l'emploi (AB) ainsi obtenue présente une valeur de pH comprise entre 7 et 8.

2. Produit selon la revendication 1, caractérisé en ce que la composition (A) présente une valeur de pH comprise entre 4,5 et 6,5.

3. Produit selon la revendication 1, caractérisé en ce que la composition (B) présente une valeur de pH comprise entre 8 et 9,5.

4. Produit selon l'une quelconque des revendications précédentes, caractérisé en ce que la composition (AB) prête à l'emploi, à appliquer sur le cheveu, obtenue après le mélange des compositions (A) et (B), présente une valeur de pH comprise entre 7,3 et 7,7.

5. Produit selon l'une quelconque des revendications précédentes, caractérisé en ce qu'il contient comme aminoacide la glycine.

6. Produit selon une ou plusieurs des revendications 1 à 5, caractérisé en ce qu'il contient au moins un sel ferreux à une concentration telle que la teneur en ions ferreux soit comprise entre 1 et 100 ppm.

7. Produit selon la revendication 6, caractérisé en ce que la teneur en ions ferreux est comprise entre 2 et 50 ppm.

8. Produit selon la revendication 7, caractérisé en ce que la teneur en ions ferreux est environ comprise entre 3 à 10 ppm.
